# EUROPÄISCHE PATENTSCHRIFT

(11) **EP 0 477 466 B1**
(45) Veröffentlichungstag und Bekanntmachung des Hinweises auf die Patenterteilung: **26.06.1996**
(21) Anmeldenummer: 91107164.5
(22) Anmeldetag: 03.05.1991
(51) Int. Cl.: A61F 2/16

(54) **Vorrichtung zum Implantieren einer gefalteten Intraokularlinse aus gummielastischem Material, insbesondere Silikonlinse**
Device for implanting a folded intraocular lens of elastic rubber material particularly a silicone lens
Appareil à implanter une lentille intraoculaire pliée en matière élastique en particulier une lentille en silicone

(30) Priorität: 26.09.1990 DE 4030492
(43) Veröffentlichungstag der Anmeldung: 01.04.1992
(73) Patentinhaber: Chiron Adatomed Pharmazeutische und Medizintechnische Gesellschaft mbH, D-85609 Dornach (DE)
(72) Erfinder: Kammann, Jochen, Dr.Med., W-4600 Dortmund 1 (DE); Dretzler Ulrich, Dipl.-Ing., W-4600 Dortmund 30 (DE); Kanert, Otmar, Prof.Dr., W-4600 Dortmund 50 (DE)
(74) Vertreter: Nöth, Heinz, Dipl.-Phys.

(56) Entgegenhaltungen:
- EP-A- 0 363 213
- US-A- 4 108 182
- US-A- 4 681 102
- US-A- 4 834 094

## Beschreibung

Die Erfindung betrifft eine Vorrichtung zum Implantieren einer gefalteten Intraokularlinse aus gummielastischem Material, insbesondere Silikonlinse, mit einem röhrchenförmigen Gehäuse, einer im Gehäuse in Gehäuse-Längsrichtung geführten Schubstange, einem Drehantrieb zur Erzeugung einer Drehbewegung mit einem am einen Gehäuseende angeordneten Implantierwerkzeug, mit dem die gefaltete Linse durch eine Schnittöffnung im Auge in die Linsenkapsel des Auges einsetzbar ist, und mit einem Stößel am zum Implantierwerkzeug gekehrten Ende der Schubstange, der durch Verschieben der Schubstange in Richtung Implantierwerkzeug die Intraokularlinse vom Implantierwerkzeug trennt.

Eine Vorrichtung dieser Art ist durch die EP-A-0 363 213 bekannt. Als Drehantrieb dient bei dieser Vorrichtung ein getrennt vom röhrchenförmigen Gehäuse angeordneter und über eine elastische Welle mit dem Gehäuse gekoppelter Zahnstangentrieb. Das Implantierwerkzeug ist als Greifwerkzeug mit Backen und Zangen zum Greifen und Fassen der Intraokularlinse ausgebildet, dessen freies Ende samt gefalteter Intraokularlinse in die Schnittöffnung im Auge eingesetzt wird, wobei nach erfolgtem Einsatz in die Linsenkapsel durch Rotation der Vorrichtung die Intraokularlinse freigegeben wird, so daß diese in ihre ursprüngliche Linsenform zurückspringen kann.

Hinsichtlich ihrer Konstruktion ist diese Vorrichtung und insbesondere ihr Implantierwerkzeug aufwendig. Die zur Freigabe der Intraokularlinse in der Linsenkapsel erforderliche Rotation der Vorrichtung birgt zudem eine nicht unerhebliche Gefahr, insbesondere kann sie bei ungeübter Handhabung zur Beschädigung der Linsenkapsel führen.

Ähnliche Vorrichtungen mit Implantierwerkzeugen mit an ihren Enden speziell geformten Pinzetten für den Einsatz der gefalteten Intraokularlinse in die Linsenkapsel erweisen sich gleichfalls als mühsam in ihrer Handhabung.

Aufgabe der Erfindung ist es demgegenüber, eine Vorrichtung der eingangs genannten Art so zu gestalten, daß sie beim Einsetzen der gefalteten Intraokularlinse in die Linsenkapsel bei einer Kataraktoperation sicher gehandhabt werden kann und unter Vermeidung jeglicher Rotation des Implantierwerkzeugs und damit unter Vermeidung jeglicher Gefahr einer Schädigung des Auges, insbesondere der Linsenkapsel arbeitet.

Die Aufgabe wird erfindungsgemäß dadurch gelöst, daß als Drehantrieb im Gehäuse ein Spindel-Mutter-Trieb mit Spindel, Kugelmutter und Hülse angeordnet ist, dessen Drehbewegung auf die in der Kugelmutter geführte Spindel übertragbar ist, wobei die Kugelmutter mit der Hülse verschraubt und die Hülse mittels Führungselementen im Gehäuse linear geführt ist, so daß die Drehbewegung der Spindel in eine lineare Bewegung der Hülse umgewandelt wird, daß der Hülse zur Übertragung ihrer linearen Bewegung auf die Schubstange eine Kupplung, z.B. eine Helix-Kupplung nachgeordnet ist und daß das Implantierwerkzeug röhrchenförmig ist und einen an die gefaltete Linse angepaßten Innendurchmesser aufweist.

Durch diese Vorrichtung wird ein sicheres Halten der gefalteten Linse aus gummielastischem Material im Inneren des röhrchenförmigen Implantierwerkzeugs gewährleistet. Ferner wird gewährleistet, daß die gefaltete Linse unter Ausschluß jeglicher Drehmomenteinwirkung auf sie einwandfrei und mit gezielter Führung in die Linsenkapsel eingesetzt wird, so daß sie sich dort entfalten kann und die gewünschte Stellung nach dem Entfalten in die ursprüngliche Linsenform in der Linsenkapsel einnimmt. Das Einsetzen der gefalteten Linse läßt sich in der Weise bewerkstelligen, daß nach dem Auffalten der Linse der Linsenäquator mit dem Äquator der Linsenkapsel übereinstimmt.

Für unterschiedliche Stärken und Durchmesser der Intraokularlinsen können unterschiedliche Querschnitte und Querschnittsformen des Röhrcheninneren des Implantierwerkzeugs vorgesehen werden. In bevorzugter Weise kann das Implantierwerkzeug als sterilisierbarer Aufbewahrungsbehälter für die gefaltete Linse ausgebildet sein.

Mit Hilfe eines Adaptereinsatzes, beispielsweise durch Arretierverschluß und/oder Schraubverschluß kann das Implantierwerkzeug in vorteilhafter Weise am Gehäuse des auch als Manipulator bezeichneten restlichen Teils der Vorrichtung befestigt werden. Der Adaptereinsatz kann hierfür ebenfalls an die unterschiedlichen Querschnitte und Querschnittsformen des Implantierwerkzeugs angepaßt sein.

Der Stößel kann hierzu in dem Adaptereinsatz angeordnet sein oder in dem röhrchenförmigen, insbesondere als Aufbewahrungsbehälter für die gefaltete Linse dienenden Implantierwerkzeug angeordnet sein.

Eine gleichmäßige Schubbewegung der exakt im Gehäuse des Manipulators geführten Schubstange, beispielsweise mit einer ziemlich konstanten Vorschubgeschwindigkeit, die maximal 1 mm pro Sekunde betragen kann, erreicht man mit Hilfe eines Drehantriebs, der insbesondere ein in seiner Drehzahl regelbarer Gleichspannungs-Mikromotor sein kann. Die Drehbewegung des Drehantriebs wird über die im Manipulator, insbesondere zwischen Hülse und Schubstange angeordnete Kupplung in eine lineare bzw. Längsbewegung umgesetzt, die auf die Schubstange übertragen wird.

Auf diese Weise wird ein exaktes Einsetzen der gefalteten Linse bei einfacher Handhabung erreicht.

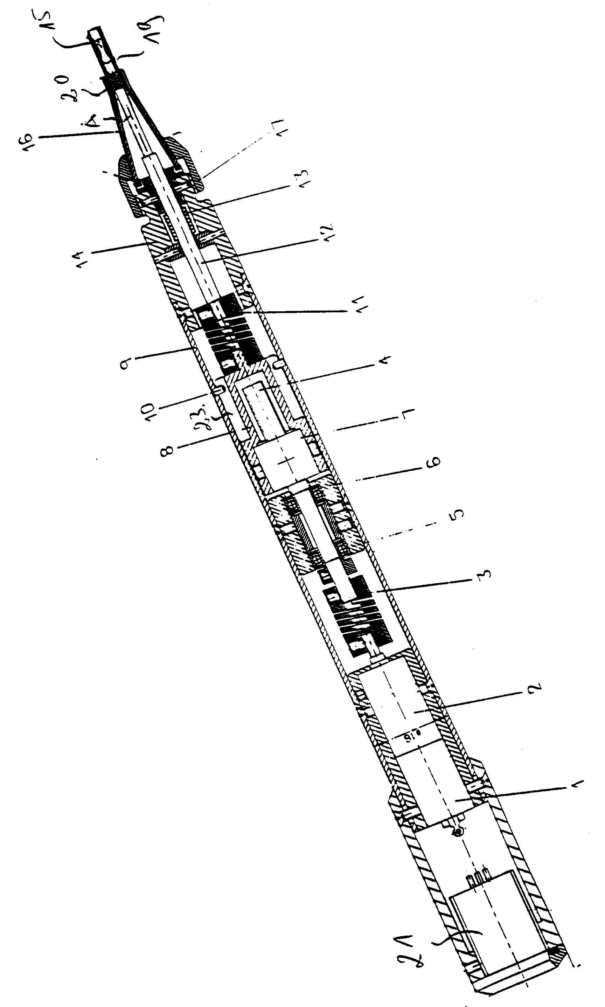

In der Figur ist in schnittbildlicher Darstellung ein Ausführungsbeispiel der Erfindung dargestellt. Anhand dieser Figur wird die Erfindung noch näher erläutert.

Die Figur zeigt in einem Längsschnitt ein Ausführungsbeispiel für die erfindungsgemäße Vorrichtung zum Implantieren einer gefalteten Intraokularlinse 15. Die Intraokularlinse 15 befindet sich in gefaltetem Zustand in einem röhrchenförmigen Implantierwerkzeug 19. Der Innendurchmesser dieses röhrchenförmigen Implantierwerkzeugs 19 ist an die Stärke und den Durchmesser bzw. die Querschnittsform der gefalteten Intraokularlinse 15, die insbesondere eine Silikonlinse ist, angepaßt. Das röhrchenförmige Implantationswerkzeug ist in einem kegelförmigen Adaptereinsatz 16 eingesetzt. Dieser Adaptereinsatz 16 kann mit der im einzelnen noch näher zu erläuternden Vorrichtung, die quasi als Manipulator bezeichnet werden kann, verbunden werden. Hierzu besitzt der Adaptereinsatz 16 zwei Klauen 18, die in einem Gehäusekopf 14 des rohrförmigen Gehäuses 9 der Vorrichtung in ihrer Lage fixiert arretierbar sind. Mit Hilfe einer aufgesetzten Mutter 17 erfolgt dann die endgültige Fixierung des kegelförmigen Adaptereinsatzes 16 am Gehäuse 9.

Im Gehäusekopf 14 ist in Längsrichtung des Implantierwerkzeugs 19, d.h. entlang einer gemeinsamen Achse A eine Schubeinrichtung, bestehend aus einer Schubstange 12 und einem Stößel 20, geführt. Die Schubstange 12 wird über ein Miniaturkugellager 13 exakt im Gehäusekopf 14 in Längsrichtung, d.h. in Richtung der Achse A, geführt. Am vorderen freien ende der Schubstange befindet sich ein Stößel 20, der im kegelförmigen Adaptereinsatz 16 oder auch im Implantierwerkzeug 19 vorgesehen sein kann. Dieser Stößel, welcher an den Querschnitt des Röhrcheninneren des Implantierwerkzeugs 19 angepaßt ist, besteht aus einem weichen Material, damit beim Verschieben der gefalteten Intraokularlinse 15 im Implantierwerkzeug 19 eine schonende Behandlung gewährleistet wird. Die Stange 12, der Stößel 20 und die gefaltete Linse 15 sind längs der Achse A zueinander ausgerichtet.

Um die Vorschubbewegung der Schubstange 12 zu erhalten, besitzt der Manipulator einen Drehantrieb, der insbesondere als Elektromotor und beim dargestellten Ausführungsbeispiel als ein Drehzahl regelbarer Gleichspannungsmikromotor 1 ausgebildet ist. Mit Hilfe einer Batterie 21 kann der Mikromotor gespeist werden.

An den Mikromotor 1 ist ein Planetengetriebe 2 gekoppelt. Die Drehbewegung des Mikromotors 1 wird ferner über eine an das Planetengetriebe 2 angeschlossene Kupplung 3, welche bevorzugt als Helix-Kupplung ausgebildet ist, an eine Spindel 4, die beim Ausführungsbeispiel als Miniatur-Kugelrollspindel ausgeführt ist, weitergeleitet, so daß die Spindel 4 in Drehung versetzt wird.

Die Spindel 4 ist über zwei Kugellager 5, insbesondere Radial-Rillenkugellager, in einem Lagergehäuse 6 fliegend gelagert und in axialer Richtung gesichert. Das Lagergehäuse 6 ist am rohrförmigen Manipulatorgehäuse 9 festgeschraubt.

Zur Umwandlung der Drehbewegung der Spindel 4 in eine Längsbewegung ist die Spindel 4 Bestandteil eines Spindel-Mutter-Triebs. Die Spindel 4 ist hierzu in einer Kugelmutter 7 des Spindel-Mutter-Triebs geführt. Die Kugelmutter 7 ist mit einer Hülse 8 verschraubt. Die Hülse 8 ist am rohrförmigen Manipulatorgehäuse 9 mit Hilfe von Stiften 10 längsgeführt. Die Stifte 10 ragen in das Innere des rohrförmigen Gehäuses 9. Die Drehbewegung der Spindel 4, welche beim dargestellten Ausführungsbeispiel bevorzugt als Kugelrollspindel ausgebildet ist, wird in bekannter Weise in einem Innengewinde der Kugelmutter 7 geführt, so daß die Drehung der Spindel 4 in eine Längsbewegung der Kugelmutter 7 umgesetzt wird. Die Längsbewegung der Kugelmutter 7 wird auf die Hülse 8 übertragen, die in der beschriebenen Weise mit Hilfe der festeingesetzten Stifte 10 im Innern des rohrförmigen Manipulatorgehäuses in Längsrichtung, d.h. in Richtung der Achse A, geführt ist. Hierbei gleiten die festeingesetzten Stifte 10 in Längsnuten 23 der Hülse 8.

Die lineare Bewegung in axialer Richtung A der Hülse 8 wird über eine weitere Kupplung 11, die ebenfalls in bevorzugter Weise als Helix-Kupplung ausgebildet ist, auf die Schubstange 12, welche über das Miniaturkugellager 13 im Gehäusekopf 14 gelagert ist, übertragen.

Die Schubstange 12 kann mit einer maximalen Vorschubgeschwindigkeit von etwa 1 mm/sec in Vorwärtsrichtung bewegt werden, so daß dabei die gefaltete Intraokularlinse 15 aus dem Implantierwerkzeug 19 in die Linsenkapsel eingeschoben wird.

Die Rückwärtsbewegung der Schubstange 12 läßt sich durch Drehrichtungsänderung des Gleichspannungsmikromotors 1 herbeiführen. Die Schubstange 12 wird dabei wieder in ihre Ausgangsposition zurückgestellt.

Damit der Manipulator dampfsterilisiert werden kann, sind in bevorzugter Weise sämtliche Durchführungen O-Ring-gedichtet.

Die gesamte Längsausdehnung der in der Figur dargestellten Vorrichtung beträgt etwa 215 mm. Die aus dem Adaptereinsatz 16 herausragende Länge des Implantierwerkzeugs 19 beträgt etwa 10 mm. Mithin ist auch der Vorschub S der Vorschubstange 12 auf etwa 10 mm bemessen. Die Länge des auf den Manipulator aufgesetzten Adaptereinsatzes 16 zusammen mit dem Implantierwerkzeug 19 beträgt etwa 27 mm. Der Durchmesser des Manipulators beträgt im Bereich der Batterie 21 etwa 20 mm.

## Patentansprüche

1. Vorrichtung zum Implantieren einer gefalteten Intraokularlinse aus gummielastischem Material, insbesondere Silikonlinse, mit einem röhrchenförmigen Gehäuse (9) mit einer im Gehäuse in Gehäuse-Längsrichtung geführten Schubstange (12), einem Drehantrieb zur Erzeugung einer Drehbewegung, einem am einen Gehäuseende angeordneten Implantierwerkzeug (19), mit dem die gefaltete Linse durch eine Schnittöffnung im Auge in die Linsenkapsel des Auges einsetzbar ist, und mit einem Stößel (20) am zum Implantierwerkzeug gekehrten Ende der Schubstange (12), der durch Verschieben der Schubstange (12) in Richtung Implantierwerkzeug (19) die Intraokularlinse vom Implantierwerkzeug (13) trennt,
dadurch **gekennzeichnet,**
daß als Drehantrieb im Gehäuse (9) ein Spindel-Mutter-Trieb (22) mit Spindel (4), Kugelmutter (7) und Hülse (8) angeordnet ist, dessen Drehbewegung auf die in der Kugelmutter (7) geführte Spindel (4) übertragbar ist, wobei die Kugelmutter (7) mit der Hülse (8) verschraubt und die Hülse (8) mittels Führungselementen (10, 23) im Gehäuse (9) linear geführt ist, so daß die Drehbewegung der Spindel (4) in eine lineare Bewegung der Hülse (8) umgewandelt wird, daß der Hülse (8) zur Übertragung ihrer linearen Bewegung auf die Schubstange (12) eine Kupplung (11) nachgeordnet ist und daß das Implantierwerkzeug (19) röhrchenförmig ist und einen an die gefaltete Linse (15) angepaßten Innendurchmesser aufweist.

2. Vorrichtung nach Anspruch 1, dadurch gekennzeichnet, daß der Stößel (20) dem Querschnitt des Röhrcheninneren des Implantierwerkzeugs (19) angepaßt ist.

3. Vorrichtung nach einem der Ansprüche 1 und 2, dadurch gekennzeichnet, daß das Implantierwerkzeug (19) über einen Adaptereinsatz (16) am Gehäuse (9, 14) befestigbar ist.

4. Vorrichtung nach einem der Ansprüche 1 bis 3, dadurch gekennzeichnet, daß das Implantierwerkzeug (19) als sterilisierter Aufbewahrungsbehälter für die Linse (15) ausgebildet ist.

5. Vorrichtung nach einem der Ansprüche 1 bis 4, dadurch gekennzeichnet, daß der Stößel (20) im Adaptereinsatz (16) angeordnet ist.

6. Vorrichtung nach einem der Ansprüche 1 bis 5, dadurch gekennzeichnet, daß zur linearen Führung der Hülse (8) im Gehäuse (9) am Gehäuse Stifte (10) fest eingesetzt sind, die in das Rohrinnere des Gehäuses ragen und in Längsnuten (23) der Hülse (8) gleiten.

7. Vorrichtung nach einem der Ansprüche 1 bis 6, dadurch gekennzeichnet, daß die Kupplung (11) zwischen Hülse (8) und Schubstange (12) angeordnet ist.

8. Vorrichtung nach einem der Ansprüche 1 bis 7, dadurch gekennzeichnet, daß die Kupplung (11) als Helix-Kupplung ausgebildet ist.

9. Vorrichtung nach einem der Ansprüche 1 bis 8, dadurch gekennzeichnet, daß die Vorschubgeschwindigkeit der Schubstange (12) samt Stößel (20) ca. 1 mm pro Sekunde beträgt.

10. Vorrichtung nach einem der Ansprüche 1 bis 9, dadurch gekennzeichnet, daß die Vorschubstrecke der Schubstange (12) samt Stößel (20) ca. 10 mm beträgt.

## Claims

1. An apparatus for implanting a folded intraocular lens of material having rubber-like elasticity, in particular a silicone lens, said apparatus having a tubular housing (9) with a push-rod (12) guided within said housing along the longitudinal direction of said housing, a rotary drive for generating a rotary movement, an implantation tool (19) disposed on one housing end, with which the folded lens is insertable into the lens capsule of the eye through a cut opening in the eye, and having a plunger (20) on the end of the push rod (12) facing the implantation tool for separating the intraocular lens from the implantation tool (13) by displacement of the push rod (12) in the direction towards the implantation tool (19),
characterized in that
a spindle-nut drive (22) with spindle (4), ball nut (7) and sleeve (8) is disposed in the housing (9) as the rotary drive, the rotary movement of which is transmittable to the spindle (4) guided in the ball nut (7), the ball nut (7) being screwed to the sleeve (8) and the sleeve (8) being guided linearly in the housing (9) by means of guide members (10, 23), so that the rotary movement of the spindle (4) is transformed into a linear movement of the sleeve (8), that a coupling (11) is disposed in succession to the sleeve (8) for transmitting the linear movement thereof to the push-rod (12), and that the implantation tool (19) is of tubular shape and has an inner diameter which is conformed to the folded lens (15).

2. An apparatus according to claim 1, characterized in that the plunger (20) is conformed to the cross-section of the tubular inside of the implantation tool (19).

3. An apparatus according to any one of claims 1 and 2, characterized in that the implantation tool (19) is adapted to be attached to the housing (9, 14) through an adapter insert (16).

4. An apparatus according to any one of claims 1 to 3, characterized in that the implantation tool (19) is designed as a sterilized storage receptacle for the lens (15).

5. An apparatus according to any one of claims 1 to 4, characterized in that the plunger (20) is disposed in the adapter insert (16).

6. An apparatus according to any one of claims 1 to 5, characterized in that, for the linear guidance of the sleeve (8) in the housing (9), pins (10) are firmly inserted in the housing, said pins projecting into the tubular inside of the housing and sliding in longitudinal grooves (23) of the sleeve (8).

7. An apparatus according to any one of claims 1 to 6, characterized in that the coupling (11) is disposed between sleeve (8) and push-rod (12).

8. An apparatus according to any one of claims 1 to 7, characterized in that the coupling (11) is designed as a helix coupling.

9. An apparatus according to any one of claims 1 to 8, characterized in that the advancing speed of the push rod (12) together with the plunger (20) is approx. 1 mm per second.

10. An apparatus according to any one of claims 1 to 9, characterized in that the advancement distance of the push-rod (12) together with the plunger (20) is approx. 10 mm.

## Revendications

1. Appareil pour implanter une lentille intra-oculaire pliée en matière à élasticité du type caoutchouc, en particulier une lentille en silicone, comprenant un boîtier (9) de forme tubulaire, une tige de poussée (12) guidée dans le boîtier dans la direction axiale du boîtier, une commande en rotation pour produire un mouvement de rotation, un outil d'implantation (19) prévu à une extrémité du boîtier, avec lequel la lentille pliée peut être mise en place dans l'oeil par une ouverture découpée dans l'oeil, et un poussoir (20) à l'extrémité de la tige de poussée (12) tournée vers l'outil d'implantation, qui, par coulissement de la tige de poussée (12) dans la direction de l'outil d'implantation (19), sépare la lentille intra-oculaire de l'outil d'implantation (13),
caractérisé en ce que, comme commande en rotation dans le boîtier (9), on prévoit une commande vis-écrou (22) comportant une tige de vis (4), un écrou sphérique (7) et une douille (8), dont le mouvement de rotation est transmissible à la tige de vis (4) guidée dans l'écrou sphérique (7), l'écrou sphérique (7) étant raccordé à la douille (8) et la douille (8) étant guidée linéairement dans le boîtier (9) au moyen d'éléments de guidage (10, 23), en sorte que le mouvement de rotation de la tige de vis (4) est transformé en un mouvement linéaire de la douille (8), qu'un accouplement (11) est monté à la suite de la douille (8) pour transmettre son mouvement linéaire à la tige de poussée (12), et que l'outil d'implantation (19) est de forme tubulaire et présente un diamètre intérieur adapté à la lentille pliée (15).

2. Appareil selon la revendication 1, caractérisé en ce que le poussoir (20) est adapté à la section de l'intérieur tubulaire de l'outil d'implantation (19).

3. Appareil selon l'une des revendications 1 et 2, caractérisé en ce que l'outil d'implantation (19) peut être fixé sur le boîtier (9, 14) par l'intermédiaire d'une pièce adaptatrice (16).

4. Appareil selon l'une des revendications 1 à 3, caractérisé en ce que l'outil d'implantation (19) est réalisé sous la forme d'un récipient stérilisable pour la conservation de la lentille (15).

5. Appareil selon l'une des revendications 1 à 4, caractérisé en ce que le poussoir (20) est prévu dans la pièce adaptatrice (16).

6. Appareil selon l'une des revendications 1 à 5, caractérisé en ce que, pour assurer le guidage linéaire de la douille (8) dans le boîtier (9), des ergots (10) sont montés fixes dans le boîtier, lesquels font saillie vers l'intérieur tubulaire du boîtier et glissent dans des rainures longitudinales (23) de la douille (8).

7. Appareil selon l'une des revendications 1 à 6, caractérisé en ce que l'accouplement (11) est prévu entre la douille (8) et la tige de poussée (12).

8. Appareil selon l'une des revendications 1 à 7, caractérisé en ce que l'accouplement (11) est réalisé sous la forme d'un accouplement en hélice.

9. Appareil selon l'une des revendications 1 à 8, caractérisé en ce que la vitesse de translation de la tige de poussée (12) avec le poussoir (20) atteint environ 1 mm/seconde.

10. Appareil selon l'une des revendications 1 à 9, caractérisé en ce que l'amplitude d'avance de la tige de poussée (12) avec le poussoir (20) atteint environ 10 mm.
